# EUROPEAN PATENT SPECIFICATION

(11) **EP 2 345 441 B1**
(45) Date of publication and mention of the grant of the patent: **21.11.2018**
(21) Application number: 09819127.3
(22) Date of filing: 02.10.2009
(51) Int. Cl.: A61M 5/145

(54) **SYRINGE PUMP**
SPRITZENPUMPE
POMPE DE SERINGUE

(30) Priority: 06.10.2008 JP 2008259491
(43) Date of publication of application: 20.07.2011
(73) Proprietor: Terumo Kabushiki Kaisha, Tokyo 151-0072 (JP)
(72) Inventor: HASEGAWA Eiji, Fujinomiya-shi Shizuoka 418-0015 (JP)
(74) Representative: Gendron, Vincent Christian
(86) International application number: PCT/JP2009/067208
(87) International publication number: WO 2010/041592

(56) References cited:
- EP-A1- 1 362 606
- EP-A1- 1 462 134
- WO-A1-2006/016716
- WO-A2-96/40322
- WO-A2-03/099355
- JP-A- 7 245 966
- JP-A- 2003 070 909
- JP-A- 2004 073 373
- JP-A- 2006 243 160
- JP-A- 2006 243 160
- JP-A- 2007 306 990
- JP-A- 2007 306 990
- US-A- 5 254 096
- US-B1- 6 269 340
- US-B1- 6 750 878

## Description

### Technical Field

The present invention relates to a syringe pump in which any of various syringes is mounted and fixed, and a plunger flange of a plunger of the mounted syringe is pushed out by a slider, thereby feeding a liquid.

### Background Art

Syringe pumps are widely used in feeding a medicinal liquid or a nutritional supplement or the like to a patient accurately and for a long time, in an intensive-care unit (ICU) or the like. A syringe pump is so designed that any of various syringe is mounted and fixed thereon, and a plunger flange of a plunger of the mounted syringe is pushed out by a slider with accurate speed control, whereby high-accuracy liquid feed can be achieved.

The syringe pump is high in general purpose properties because it is so configured that variously sized syringes can be mounted thereon and the liquid feed rate can be easily set. Such syringe pumps are disclosed, for example, in Japanese Patent Laid-open No. 2004-73373 (US 7,153,290 B2) and Japanese Patent Laid-open No. 2007-306990.

A syringe pump, for mounting variously sized syringes thereon and performing liquid feed, is provided with a clamp which is pulled downward by an elastic member and contacts an upper surface of the mounted outer cylinder to thereby fix the outer cylinder, and levers for holding onto a slider the plunger flange of the plunger of the mounted syringe. The clamp can be selectively rotated into either of a release position and a fixing position, and it is possible, by elastically lowering the clamp in the fixing position, to clamp the outer cylinder. The levers are provided in a pair which can be elastically opened and closed, so that any of plunger flanges of various diameters can be secured to the slider by clamping it with the levers.

### Summary of the Invention

In a syringe pump, stable liquid feeding can be performed by securely fixing the outer cylinder and the plunger flange with the clamp and the slider having the levers as above-mentioned.

On the other hand, in a preceding stage, the operations of mounting of the outer cylinder onto the syringe mounting part, fixation of the outer cylinder with the clamp, and fixation of the plunger flange of the plunger with the levers are operations to be conducted by the medical worker, and proper operations are carried out conscientiously. In addition, in Japanese Patent Laid-open No. 2007-306990, a configuration is adopted wherein if these operations are unsatisfactory, a predetermined alarm is issued, so as to alleviate the attention burden on the medical worker operating the syringe pump. In US 5,254,096 a syringe pump is disclosed that displays a graphical representation of a syringe with points that light up when errors occur at these points on the actual syringe. EP 1 462 134 discloses an injector with a display unit that advises the user to remove air from the cartridge. EP 1 362 606 discloses a syringe pump with a battery alarm lamp. US 6 269 340 discloses a syringe pump that performs a syringe detection when an attempt is made to start the pump and displays an alert to the user when the syringe is not detected.

However, a person who does not understand the method of mounting the syringe or the operations of the clamp and the slider provided with the levers, etc. or who is not frequently engaged in the operations cannot operate the syringe pump without referring to a manual. Besides, even for a person accustomed to the operations of the syringe pump, it is desirable that it is easier to check whether an operation has been made properly or not.

Further, in relation to the conventional syringe pumps, some problems have been found.

Firstly, while a syringe pump is provided therein with a secondary battery capable of internally supplying electric power for a certain period of time in order to ensure that, even upon stop of power supply from an external power source, a predetermined coping with the situation can be made, it is yet basically desirable to securely supply the syringe pump with electric power from an external power source.

Secondly, while detection of various unusual conditions is performed during liquid feeding by use of a syringe pump, it is desired that, upon detection of an unusual condition, the kind of the unusual condition can be easily grasped.

Thirdly, while routine inspections according to predetermined standards are made compulsory in application of a syringe pump, the routine inspections are to be carried out in a plurality of procedures for a plurality of items, so that reference to a manual is substantially required for carrying out the inspections, which is burdensome for the operator.

Accordingly, it is an object of the present invention to provide a syringe pump further enhanced in operationality, particularly, a syringe pump which ensures that at the time of setting a syringe thereon, it can be easily grasped what operation is to be carried out.

The invention has been defined in claim 1. There is provided a syringe pump including: a syringe mounting part on which to mount an outer cylinder of a syringe; a slider for holding a plunger of the syringe mounted on the syringe mounting part; a clamp which contacts an upper surface of the outer cylinder mounted on the syringe mounting part and which thereby fixes the outer cylinder; a display unit for displaying predetermined information; and a control unit for controlling the display of the display unit; wherein in regard of an operation of mounting the outer cylinder onto the syringe mounting part, an operation of holding the plunger by the slider and an operation of fixing the outer cylinder by the clamp, the display unit under control of the control unit displays graphics showing respective changes between the states before and after the operations.

The display with the graphics showing respective changes between the states before and after the operations as above-mentioned makes it possible to easily grasp what operation is to be carried out in setting the syringe.

The display unit may display graphics showing respective states before and after the operations, alternately and repeatedly at a predetermined interval. This ensures that the differences between the states before and after the operations can be grasped clearly and easily, and what operation is to be carried out can be understood more easily.

The syringe pump may further include an outer cylinder detecting part for detecting that the outer cylinder is mounted on the syringe mounting part; a plunger detecting part for detecting that the plunger is held by the slider; and a clamp detecting part for detecting that the outer cylinder is fixed by the clamp; and the syringe pump may be so configured that the control unit is connected to the outer cylinder detecting part, the plunger detecting part and the clamp detecting part; and when detection information from the outer cylinder detecting part, the plunger detecting part, and the clamp detecting part is inputted to the control unit, the display unit displays a graphic showing the state after completion of the corresponding operation. Accordingly, it is possible to confirm that each operation is carried out properly by respective procedures.

In addition, there is provided a syringe pump including: a syringe mounting part on which to mount an outer cylinder of a syringe; a slider for holding a plunger of the syringe mounted on the syringe mounting part; a clamp which contacts an upper surface of the outer cylinder mounted on the syringe mounting part and which thereby fixes the outer cylinder; an outer cylinder detecting part for detecting that the outer cylinder is mounted on the syringe mounting part; a plunger detecting part for detecting that the plunger is held by the slider; a clamp detecting part for detecting that the outer cylinder is fixed by the clamp; a display unit for displaying predetermined information; and a control unit for controlling the display of the display unit; wherein the display unit under control of the control unit displays states detected respectively by the outer cylinder detecting part, the plunger detecting part, and the clamp detecting part.

This ensures that checking of the statuses of detection at the detecting parts is facilitated, and the part(s) which has not yet been mounted or has been mounted improperly can be recognized, so that operationality of the syringe pump is further enhanced.

The syringe pump as just-mentioned may be so configured that when pieces of detection information from the outer cylinder detecting part, the plunger detecting part, and the clamp detecting part are inputted to the control unit, the display unit displays graphics showing respective states after completion of an operation of mounting the outer cylinder on the syringe mounting part, an operation of holding the plunger by the slider, and an operation of fixing the outer cylinder with the clamp, which correspond respectively to the pieces of detection information. Thus, when all the works for setting the syringe have been finished, it is clearly shown that all the operations have been finished correctly, whereby checking of whether the operations have been carried out properly or not can be made easier. Accordingly, the operator's sense of security is enhanced.

A configuration wherein the control unit, upon deciding that an unusual condition is generated, displays information indicative of the kind of the unusual condition on the display unit permits the operator to easily grasp the kind of the unusual condition.

In this case, it is recommendable to adopt a configuration wherein after all of pieces of detection information from the outer cylinder detecting part, the plunger detecting part, and the clamp detecting part are inputted to the control unit and when at least one of the pieces of detection information is changed to an undetection information, the display unit displays information indicative of the kind of an unusual condition corresponding to the change.

A configuration wherein the display unit has a backlight capable of being turned on and off, and causes the backlight to blink when it is decided by the control unit that an unusual condition is generated provides a mentally attention-exciting effect on the operator.

The syringe pump as above may further include: a power source terminal through which external electric power is supplied and fed to each component; a secondary battery which is charged through the power source terminal and is capable of feeding electric power to each component; and an external power source detecting part for detecting the state of connection of external electric power to the power source terminal; and the control unit may display on the display unit information for prompting for connection of the external power source when it is decided, based on a signal from the external power source detecting part, that the external power source is not being connected. This configuration wherein the external power source detecting part is provided and the information prompting for connection of an external power source is displayed on the display unit when it is decided that external electric power is not being supplied ensures that a situation where the syringe pump is left in the state of being supplied with electric power from the secondary battery can be prevented more securely, and electric power is assuredly supplied from an external power source.

At the time of performing a prescribed inspection operation, the control unit may display on the display unit specific operations according to a procedure for the inspection operation. This permits the operator to carry out the inspection operation without referring to a manual, whereby burden on the operator is alleviated.

According to the syringe pump of the present invention, operations to be conducted are displayed with graphics showing the changes between the respective states before and after the operations, whereby it is made possible to easily grasp what operation is to be carried out in setting the syringe. In addition, the part where the syringe has not been set properly can be easily grasped.

### Brief Description of Drawings

[FIG. 1]

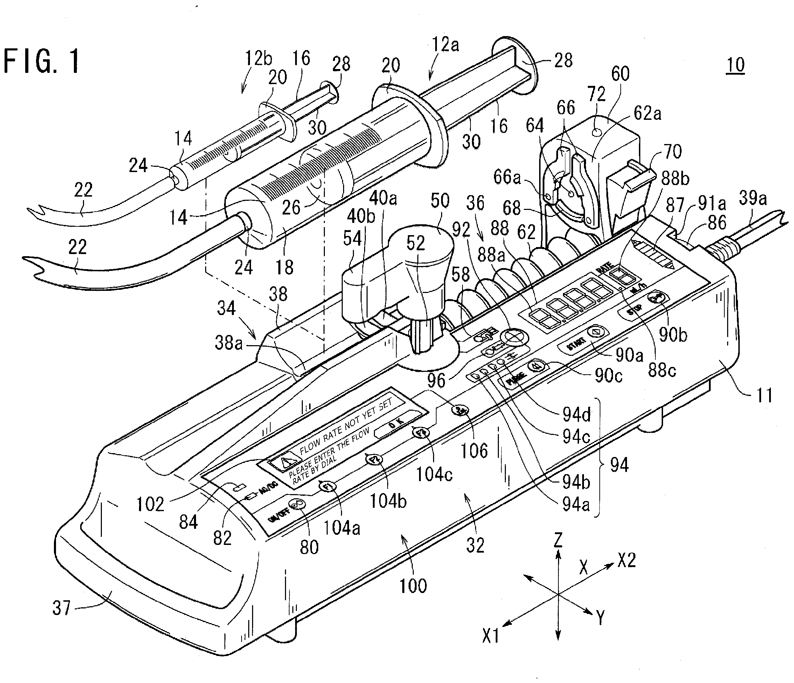

   FIG. 1 is a perspective view of a syringe pump according to the present embodiment, before a syringe is mounted.
[FIG. 2]

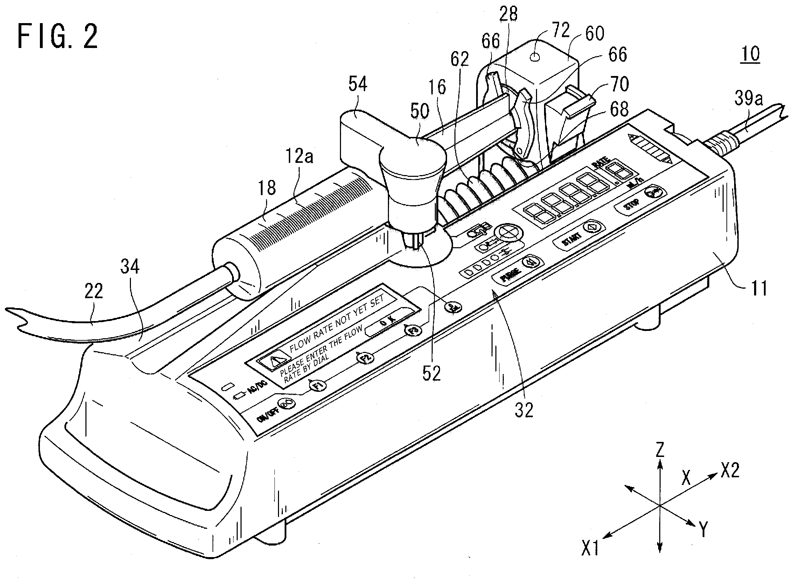

   FIG. 2 is a perspective view of the syringe pump according to the present embodiment, after the syringe is mounted.
[FIG. 3]

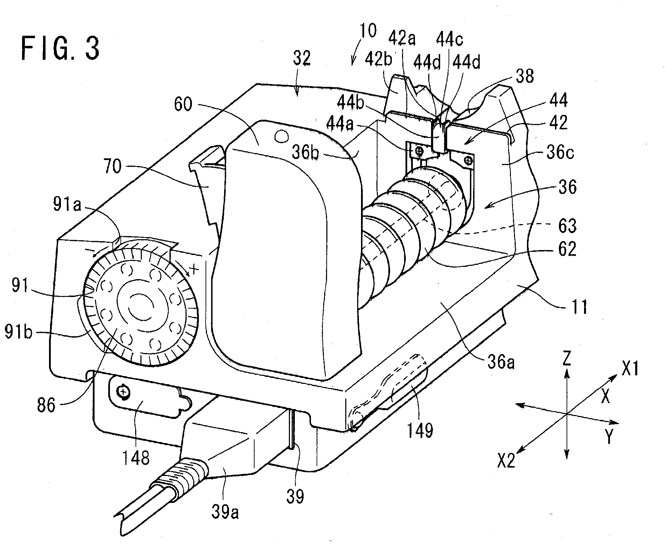

   FIG. 3 is a perspective view, as viewed from the rear side, of the syringe pump according to the present embodiment.
[FIG. 4]

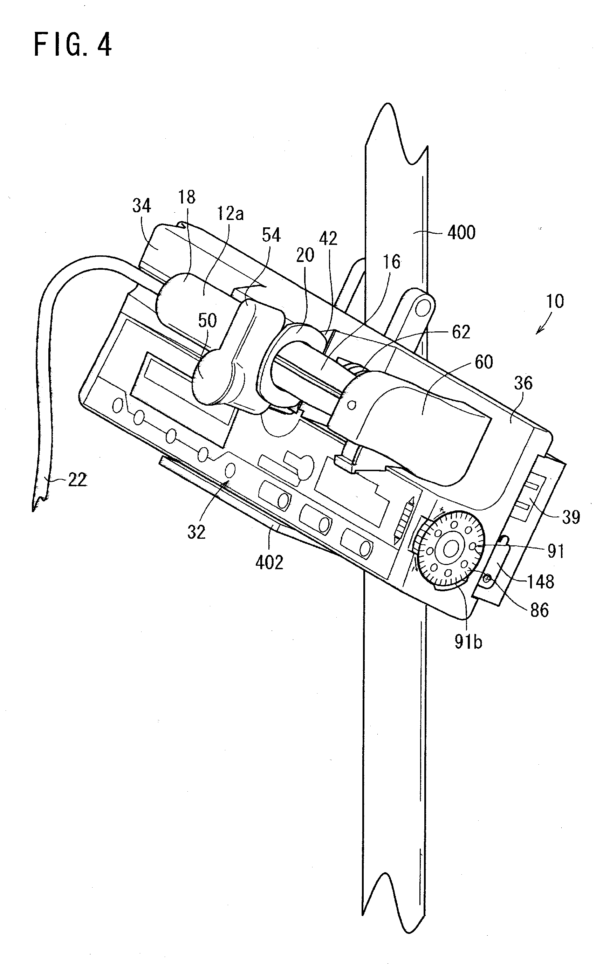

   FIG. 4 is a perspective view of the syringe pump set obliquely.
[FIG. 5]

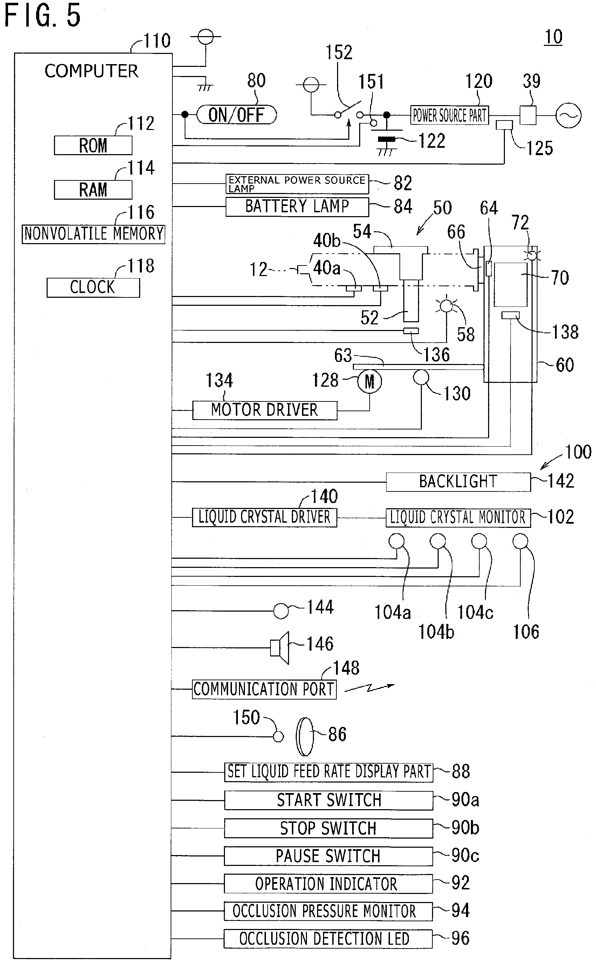

   FIG. 5 is a block diagram of the syringe pump according to the present embodiment.
[FIG. 6]

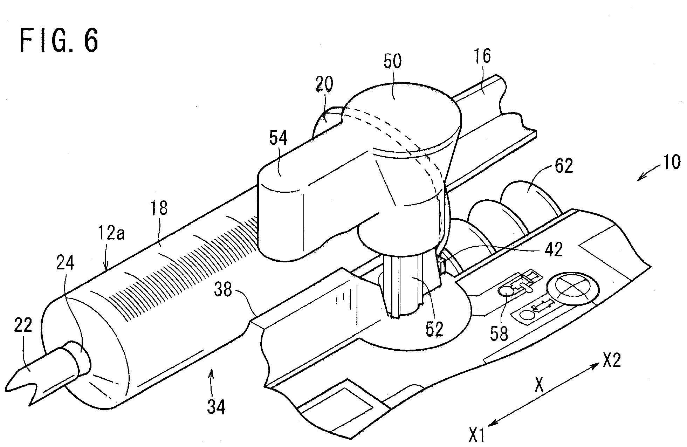

   FIG. 6 illustrates the manner in which to carry out a-operation of setting a syringe.
[FIG. 7]

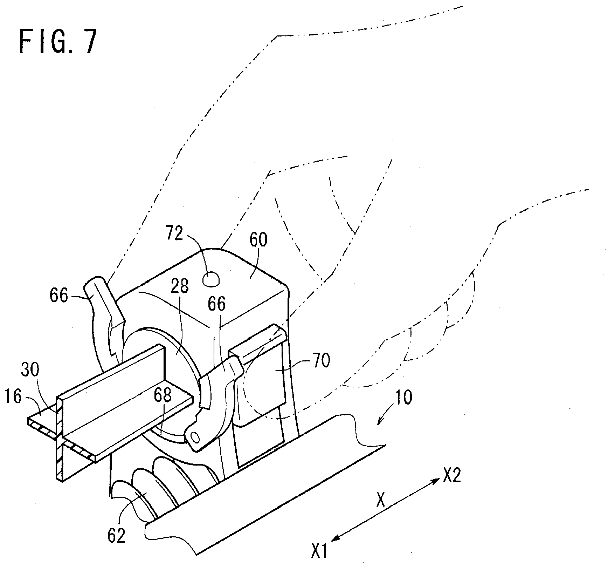

   FIG. 7 illustrates the manner in which to carry out b-operation of setting the syringe.
[FIG. 8]

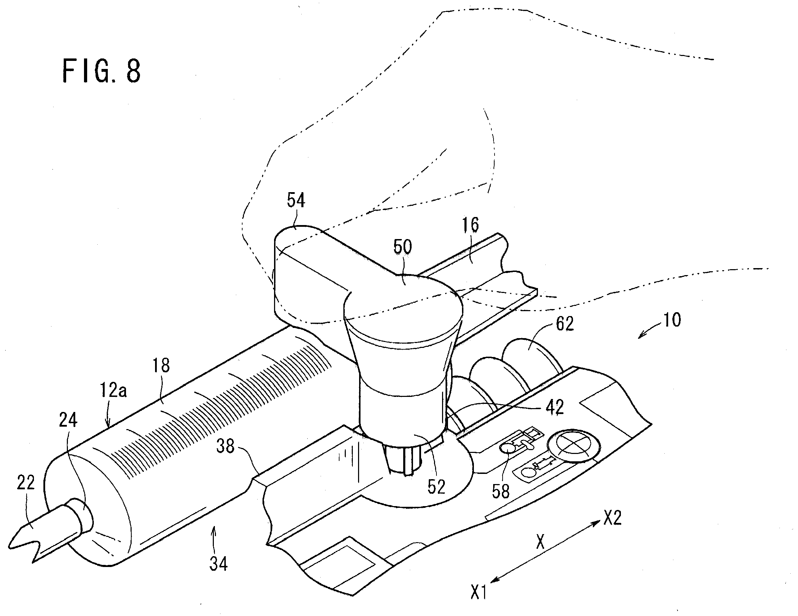

   FIG. 8 illustrates the manner in which to carry out c-operation of setting the syringe.
[FIG. 9]

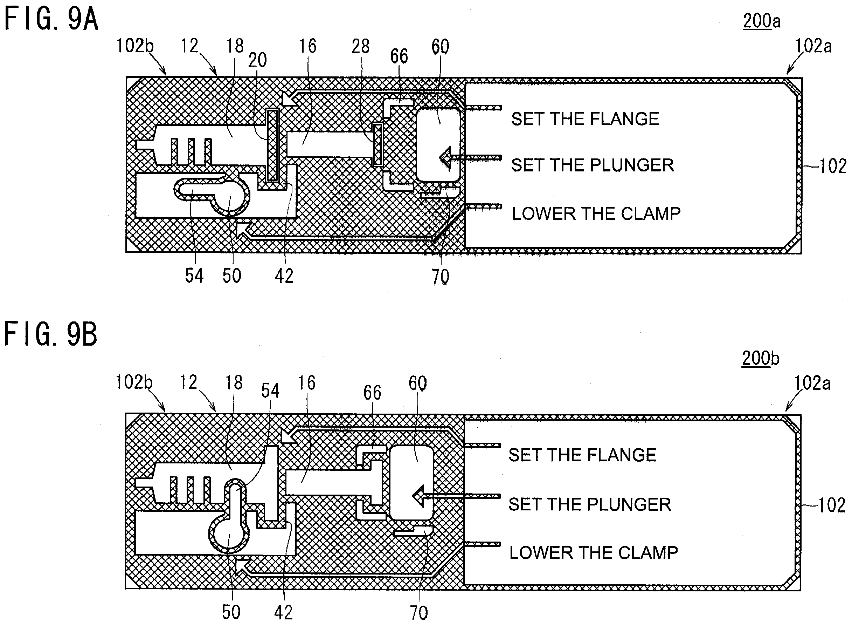

   FIG. 9A shows an initial state screen, and FIG. 9B shows a first syringe normal mount state screen.
[FIG. 10]

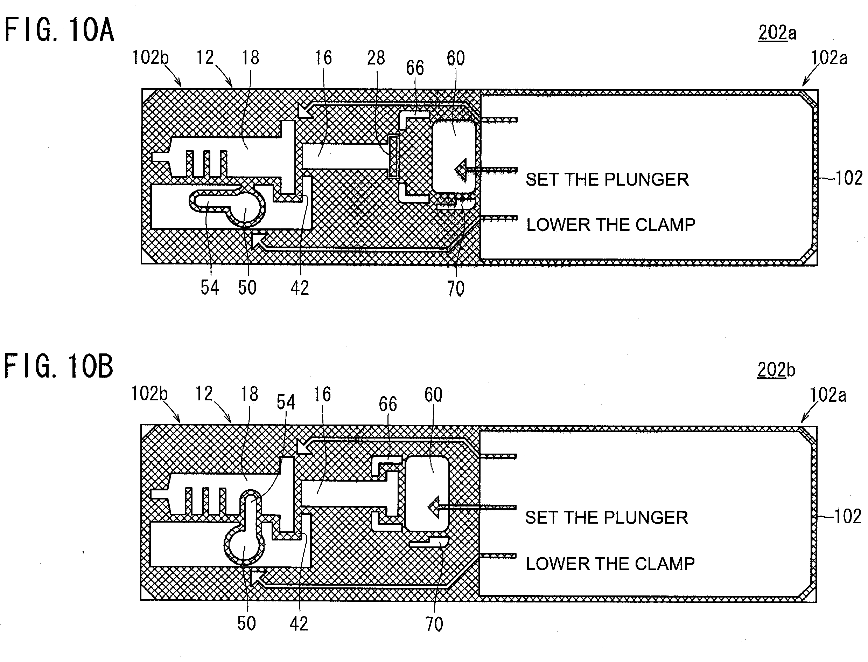

   FIG. 10A shows a flange set state screen, and FIG. 10B shows a second syringe normal mount state screen.
[FIG. 11]

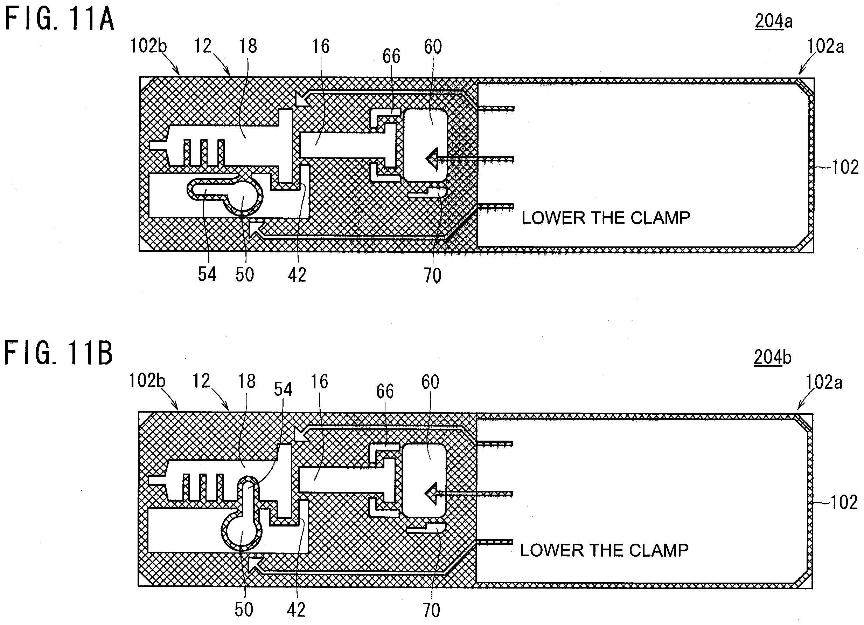

   FIG. 11A shows a slider set state screen, and FIG. 11B shows a third syringe normal mount state screen.
[FIG. 12]

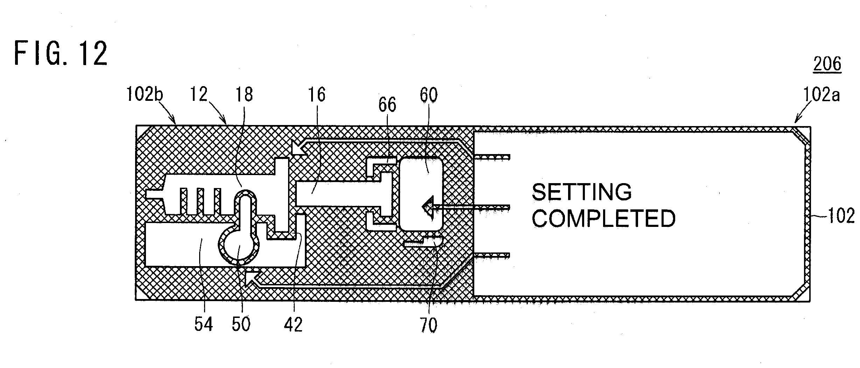

   FIG. 12 shows a setting-completed screen.
[FIG. 13]

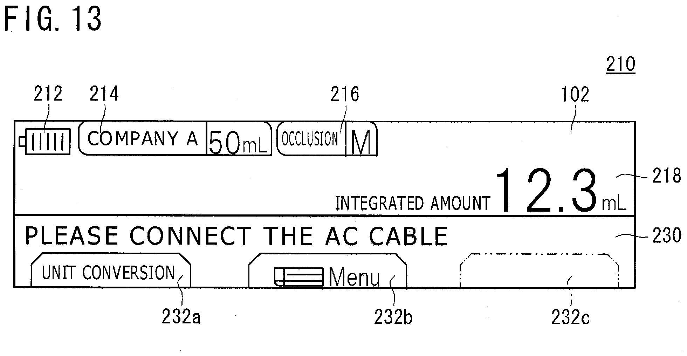

   FIG. 13 shows an operation screen.
[FIG. 14]

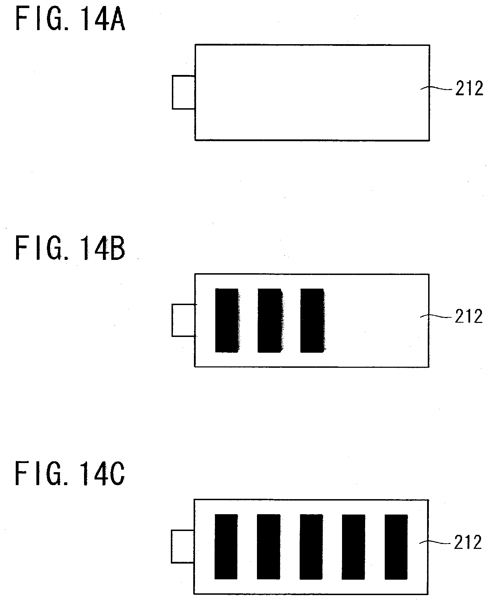

   FIG. 14A shows a power source symbol at the time when a battery is completely discharged, FIG. 14B shows a power source symbol at the time when the battery charge amount is roughly half the full charge, and FIG. 14C shows a power source symbol at the time when the battery is fully charged.
[FIG. 15]

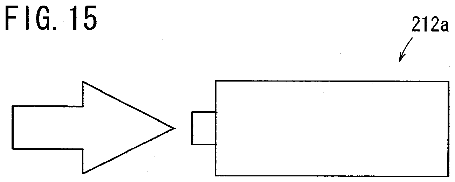

   FIG. 15 shows a symbol representing that the battery is being charged.
[FIG. 16]

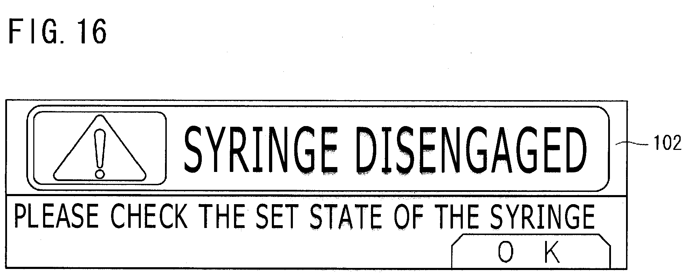

   FIG. 16 shows a screen at the time when an alarm is issued.
[FIG. 17]

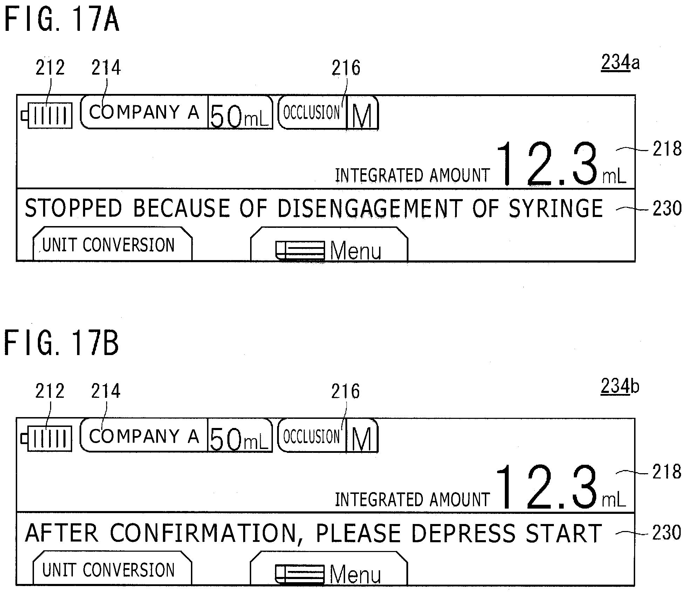

   FIG. 17A shows a screen after disposal of a first unusual condition, and FIG. 17B shows a screen after disposal of a second unusual condition.
[FIG. 18]

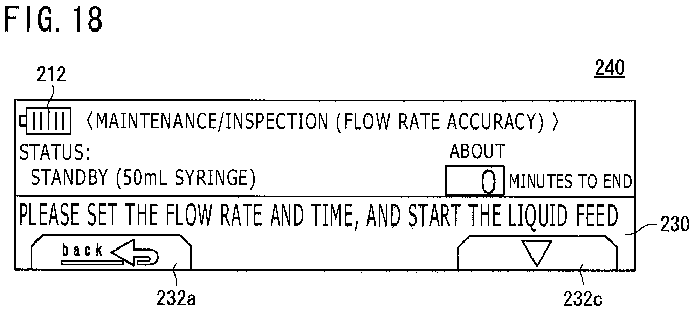

   FIG. 18 shows a screen at the time of an inspection operation.

### Mode for Carrying Out the Invention

Now, a syringe pump according to the present invention will be described below taking an embodiment thereof and referring to the accompanying drawings, namely, FIGS. 1 to 18. First, a syringe 12a to be applied to a syringe pump 10 according to the present embodiment will be described.

As shown in FIG. 1, the syringe 12a is a general-use one that has been conventionally used, which includes a syringe outer cylinder (outer cylinder) 18 and a plunger 16. The syringe outer cylinder 18 has a transparent syringe body 14 to be filled with a medicinal liquid or the like, an outer cylinder flange 20 provided on the proximal side (base end side), and a discharge port 24 which is provided on the distal side (tip side) and to which a tube 22 is connected. The outer cylinder flange 20 is substantially elliptic in shape. Of the outer cylinder flange 20, the width in the minor diameter direction is slightly larger than the diameter of the body 14, and the width in the major diameter direction is set to such a value that the user's fingers can be put thereon. The outer cylinder flange 20 is appropriately thin; for example, the thickness is about 1 to 2 mm. The tube 22 is connected at its one end to the discharge port 24, and at its other end to an indwelling needle or the like (not shown), so that a medicinal liquid can be injected into a patient therethrough.

The plunger 16 has a gasket 26 which is provided at the distal end and by which the medicinal liquid in the syringe outer cylinder 18 is fed out, a disk-shaped plunger flange 28 provided at the proximal end, and a rib 30 interconnecting the gasket 26 and the plunger flange 28. The plunger flange 28 has such an area that it can be pushed by the user's thumb, and is appropriately thin; for example, the thickness is about 1 to 2 mm. The rib 30 is cross-shaped in section, with a radial size greater on the distal side and smaller on the proximal side, and has sufficient rigidity and a light weight. A plurality of different-sized syringes (for instance, 5 mL type, 10 mL type, 20 mL type, 30 mL type, 50 mL type, and 100 mL type) are applicable to the syringe pump 10, and the syringe 12a is of the longest and largest-in-diameter type (namely, of the highest-capacity type, for example 100 mL type). A syringe 12b also shown in FIG. 1 is of the shortest and smallest-in-diameter type (namely, of the lowest-capacity type, for example, 5 mL type). The syringes applicable to the syringe pump 10 will be referred to also as syringe 12 in a representative manner.

In the description of the syringe pump 10 as follows, the major width direction will be referred to as X-direction, the minor width direction as Y-direction, and the height direction as Z-direction. If necessary, taking FIG. 1 as a reference, the left side in regard of X-direction will be referred to also as Xl-direction, and the right side as X2-direction.

As shown in FIGS. 1 to 3, the syringe pump 10 according to the present embodiment has a configuration in which a box-shaped housing 11 slightly elongated in the X-direction serves as a base. The syringe pump 10 has an operating part 32 constituting a roughly lower half thereof in plan view (the plan view is omitted, since it can be recognized from FIG. 1), a syringe mounting part 34 constituting a roughly left half of the upper half thereof in plan view, and a plunger driving part 36 constituting a roughly right half of the upper half thereof in plan view. The syringe pump 10 is provided with a grip 37 at a left end portion thereof and is, therefore, excellent in portability. Substantially the whole area of the upper surface of the syringe pump 10 is a roughly flat surface formed in a very gentle arched shape in regard of the depth direction. The syringe pump 10 has a highly waterproof structure in which switches (for example, tact switches) at the operating part 32 are covered with a facing sheet.

The syringe pump 10, basically, has a splash-proof structure because the medicinal liquid in the syringe 12 may drop thereon, a drip-feed solution provided on the upper side may drop thereon, or a disinfectant or the like being used in the surroundings may scattered thereon.

The syringe 10 is supplied with electric power from an external power source (AC 100 V, AC 200 V, or the like) through an AC cable 39a connected to a power source plug socket (power source terminal) 39 provided at a lower portion of a side surface thereof.

In the following description, the vertical direction will be determined by assuming that the syringe pump 10 is in the state of being put on a horizontal desktop. The orientation of the syringe pump 10 in practical use, however, is not limited to this. For example, as shown in FIG. 4, the syringe pump 10 may be used in the state of being obliquely fixed to a mounting means 402 of an infusion stand 400. The mounting means 402 can be adjusted in inclination angle and height while stably holding the syringe pump 10. In addition, electric power can be supplied to an exclusive-use terminal (not shown) other than the power source plug socket 39 from the mounting means 402, which naturally is preferable.

Returning to FIGS. 1 to 3, the syringe mounting part 34 is a part for mounting and fixing the syringe outer cylinder 18 in such an orientation that the discharge port 24 is located on the left side, and is provided with a curved part 38 having a recessed arched surface according to the diameter of the syringe outer cylinder 18 (the largest one), near than the surface of the operating part 32. The curved part 38 forms a recessed arched cylindrical surface with reference to the axial direction (X-direction) of the syringe outer cylinder 18 to be mounted thereon.

The curved part 38 is located at a roughly right half of the syringe mounting part 34, and is composed of wall parts provided on the viewer's side and the depth side, respectively. The curved part 38 has a narrow groove 38a provided in the lowermost surface to extend in the axial direction, two detection switches (syringe outer cylinder detecting parts) 40a and 40b provided in the vicinity of the X2-direction and adjacent to each other along the axial direction, a vertical-direction (in plan view) setting groove 42 which is provided in an X2-direction end portion and which holds the outer cylinder flange 20, and a spring body 44 for elastically pressing and stabilizing the outer cylinder flange 20 mounted.

The setting groove 42 is a shallow groove in which to mount the outer cylinder flange 20, and is so shaped that the largest-diameter direction of the outer cylinder flange 20 will be oriented in the vertical direction (in plan view). The setting groove 42 is composed of a first groove wall 42a constituting a part on the side of a slider 60, and a second groove wall 42b constituting a part on the side of the syringe mounting part. Since the thickness of the outer cylinder flange 20 varies depending on the manufacturer, the width of the setting groove 42 is designed with an appropriate margin; the groove width is about 3.5 mm. The setting groove 42 ensures proper positioning of the syringe outer cylinder 18 in the axial direction.

When the syringe outer cylinder 18 is mounted on the syringe mounting part 34, a claw 44c of the spring body 44 makes contact with a lowermost portion of the outer cylinder flange 20 mounted in the setting groove 42, and presses the outer cylinder flange 20 toward the side of the syringe mounting part 34 (in the Xl-direction), thereby bringing the outer cylinder flange 20 into contact with an end face of the second groove wall 42b. As a result, the syringe outer cylinder 18 is prevented from chattering in the axial direction, so that stable liquid feed is enabled. In addition, the liquid feed can be started instantaneously when pushing of the plunger 16 with the slider 60 is started. Thus, flow rate build-up performance is enhanced.

The narrow groove 38a is a groove for contriving stabilization in mounting the syringe 12b of the small-diameter type.

The two detection switches 40a and 40b are slightly projected from the lower surface of the syringe mounting part 34, and are pushed in (down) when the syringe outer cylinder 18 is mounted properly, whereby it can be detected that the syringe outer cylinder 18 has been mounted. The interval between the two detection switches 40a and 40b in the axial direction is preferably 5 to 10 mm, and the detection switches 40a and 40b are covered with a single ethylene-propylene rubber film, which ensures high water resistance and weatherability. The ethylene-propylene rubber film and the curved part 38 are preferably whitish in color, for securing good visibility of graduations printed in black on the syringe outer cylinder 18. The detection switches 40a, 40b may be provided in three or more within the range of the length of the mounted syringe outer cylinder 18 of the syringe 12 of the shortest type. The two detection switches 40a and 40b may not necessarily be disposed rectilinearly, insofar as they are disposed at different positions in the axial direction of the syringe 12 to be mounted.

As shown in FIG. 3, the spring body 44, which is made of resin, includes an attachment plate 44a attached to a side surface of the curved part 38, a spring plate part 44b projecting upward from the attachment plate 44a, the short claw 44c bent at an acute angle from the tip of the spring plate part 44b and extending obliquely downward, and a pair of thin projections (projected portions) 44d slightly projected upward on both sides of a joint portion between the spring plate part 44b and the claw 44c. The attachment plate 44a is attached to a side surface of the first groove wall 42a by screws, and the claw 44c slightly enters into the setting groove 42. The pair of projections 44d form an X-Z plane, and are mountain-shaped as viewed along the Y-direction.

Slightly on the viewer's side of the detection switches 40a and 40b, a clamp 50 which contacts and clamps an upper surface of the syringe outer cylinder 18 mounted is provided at a boundary portion between the operating part 32 and the syringe mounting part 34. The clamp 50 has a lift rod 52 projecting from an upper surface of the operating part 32, and a rotating lever 54 provided at the upper end of the lift rod 52. The rotating lever 54 is so sized and shaped as to permit easy operation thereof by hand. When the syringe 12 is not mounted, the lift rod 52 is kept pulled down by an elastic member (not shown), and the rotating lever 54 is substantially horizontally extended in the Xl-direction from the lift rod 52.

At the time of fixing the syringe 12 after the syringe outer cylinder 18 is mounted on the syringe mounting part 34, first, the clamp 50 as a whole is drawn upward by gripping the rotating lever 54, and is rotated 90° clockwise until the rotation is restricted by a predetermined stopper. Next, while gripping the rotating lever 54, the clamp as a whole is lowered by an elastic force of the elastic member, and the lower surface of the rotating lever 54 is put into elastic contact with the upper surface of the syringe outer cylinder 18, whereby the syringe outer cylinder 18 is pressed and clamped with an appropriate force (see FIGS. 2 and 8). The rotating lever 54 presses and clamps a portion near the outer cylinder flange 20, of the syringe outer cylinder 18.

The lowering amount of the lift rod 52 is detected by a clamp sensor (clamp detecting part) 136 (see FIG. 5), whereby each of the syringe 12a, 12b and the like can be identified as the object clamped by the rotating lever 54. At the time of releasing the syringe outer cylinder 18 from the clamped state, it suffices to carry out an operation reverse to the above. The clamp sensor 136 may be so designed that it can detect a rotated state of the clamp 50.

The operating part 32 is provided, in the vicinity of the clamp 50, with a clamp check LED 58 which is put from an OFF state into a blinking state when it is decided that the pair of detection switches 40a and 40b are in an OFF state or that the clamp 50 is not clamping the syringe outer cylinder 18. A symbol is drawn adjacently to the clamp check LED 58 so that the operator can conceptually understand the function of the clamp check LED 58.

The plunger driving part 36 is provided in a space defined by a step surface 36a lower than the operating part 32, a substantially vertical wall surface 36b between the step surface 36a and the operating part 32, and a substantially vertical wall surface 36c between the step surface 36 and the syringe mounting part 34. The plunger driving part 36 has the slider 60 movable in the X-direction, and a flexible and expandable bellows cylinder 62 interconnecting the slider 60 and the wall surface 36c.

The slider 60 is a box body rounded for permitting easy operation, with a bottom portion set near the step surface 36a in height and with an upper surface portion set appropriately higher than the operating surface of the operating part 32. The slider 60 has a plunger detection switch (plunger detecting part) 64 provided on a connection surface 62a on the Xl-direction side at a position for contact with the plunger flange 28 of the syringe 12, a pair of lever 66 for fixing the plunger flange 28 in contact with the connection surface 62a, an arched support base 68 provided at the connection surface 62a so as to support the plunger flange 28, and a lock lever 70 for fixing and releasing the slider 60 in a sliding direction.

The slider 60 has the function of holding the plunger flange 28 of the plunger 16 of the syringe 12 mounted by the levers 66 and pushing out the plunger 16. The plunger detection switch 64 is turned ON when contacted by the plunger flange 28.

The lock lever 70 is projected to the viewer's side from a side surface of the slider 60 when not operated, thereby fixing the slider 60. When the lock lever 70 is pushed in elastically, manual sliding of the slider 60 in the X-direction is permitted. The operational state of the lock lever 70 is detected by a lock sensor 138 (see FIG. 5).

The pair of levers 66 are in inwardly directed arched shapes symmetrical with each other about the axis of the syringe 12. Interlockedly with pushing-in of the lock lever 70, the levers 66 are tilted respectively around axes 66a provided on the lower side, to be spaced away from each other. Interlockedly with releasing of the lock lever 70, the levers 66 are elastically closed, whereby both lateral portions of the plunger flange 28 can be gripped. The levers 66 hold an outer peripheral surface and an X2-side surface of the plunger flange 28, whereby the plunger flange 28 can be prevented from being disengaged from the slider 60. The plunger flange 28 is held at three locations by the pair of levers 66 and the support base 68, to be stabilized.

The slider 60 is provided at its upper surface with a slider check LED 72. The slider check LED 72 is put from a blinking state to an OFF state when it is decided that the plunger flange 28 is fixed to the slider 60 by the levers 66 and the slider 60 is fixed, after the slider 60 is manually moved so that the plunger flange 28 makes contact with a side surface of the slider 60 to turn ON the plunger detection switch 64 and the lock lever 70 is released.

The slider 60 can be automatically advanced in the Xl-direction by a retracting movement of a driving rod 63 (see FIG. 5) provided inside the bellows cylinder 62, in the condition where the clamp check LED 58 and the slider check LED 72 are OFF (namely, the condition where the syringe 12 is mounted accurately; this condition will hereafter be referred to as syringe normal mount state).

The retraction speed of the driving rod 63 is determined based on a liquid feed rate set by a setting dial 86 and the kind of the syringe 12 detected by the clamp 50, and liquid feed is performed by pushing out the plunger 16 while accurately controlling the retraction speed.

In relation to power source function, the operating part 32 has a power source switch 80, a green external power source lamp 82, and a red battery lamp 84. The external power source lamp 82 is turned ON when it is decided by an external power source detecting part 125 (see FIG. 5) that an external power source is connected to the power source plug socket 39. The battery lamp 84 is set blinking when the residual charge amount (also called SOC (State of Charge)) of a lithium ion battery (secondary battery) 122 is lowered. When the residual charge amount of the lithium ion battery 122 is lowered, alarming by a buzzer 144 and display of characters on a liquid crystal monitor 102 are also performed. The residual charge amount of the lithium ion battery 122 is detected by a predetermined charge amount sensor 151 (see FIG. 5). With a power source switch 80 operated, a main contactor 152 (see FIG. 5) in the inside is turned ON, whereby the power source for the syringe pump 10 as a whole is turned ON.

In relation to liquid feed rate, the operating part 32 has the setting dial 86 and a set liquid feed rate display part 88. The setting dial 86 is rotatably disposed in a bottomed dial disposing hole 91 in a longitudinal-direction side surface of the syringe pump 10, and its side surface is exposed.

The dial disposing hole 91 has a vertically (in plan view) elongated notch portion 91a for exposing an upper portion of the setting dial 86 at an upper surface, and a side recess portion 91b in a tapered shape with a predetermined width broadened diametrally outward along the opening direction. When the side surface or the part exposed from the vertically (in plan view) elongated notch portion 91a of the setting dial 86 is rotated by a finger, a dial rotation sensor 150 (see FIG. 5) in the main body reacts with a magnetic member provided inside the setting dial 86, whereby the rotating direction and the rotation amount can be detected. The dial rotation sensor 150 is, for example, a Hall device. The setting dial 86 can be rotated endlessly. As for the polarity of increase/decrease of the liquid feed rate by the setting dial 86, the upward direction (clockwise direction in side view) is plus, and the downward direction (counterclockwise direction in side view) is minus, as indicated by a polarity indication symbol 87 provided in the vicinity of the setting dial 86. A polarity indication is provided also on a side surface of the main body (see FIG. 3). The liquid feed rate set by the setting dial 86 may be recorded in a nonvolatile memory 116 so as to be held even after the power source is turned OFF.

The set liquid feed rate display part 88 is provided with five 7-segment LEDs, and the set liquid feed rate [mL/h] set by use of the setting dial 86 is displayed in a maximum of five digits. The set liquid feed rate display part 88 has a four-digit integer part 88a turned ON in green, a one-digit decimal part 88b turned ON in red, and a red decimal point LED 88c disposed between them. The decimal part 88b is slightly smaller than the integer part 88a. According to this configuration, confirmation of the digit places is more facilitated by the differences in color and size as well as the clarification of the decimal point. Particularly, since the decimal part 88b and the decimal point LED 88c are in red color which has an attention-exciting effect, the fact that the part indicated by them relates to the decimal fraction is exaggerated, facilitating the confirmation of the digit places. The set liquid feed rate display part 88 is disposed in the vicinity of the setting dial 86, so that the corresponding relationship therebetween can be easily understood by the operator.

In relation to liquid feed operation, the operating part 32 has a start switch 90a, a stop switch 90b, and a fast feed switch 90c. The start switch 90a becomes effective in the syringe normal state.

The operating part 32 has an operation indicator 92, an occlusion pressure monitor 94, and a residual amount detection LED 96, as display parts related to liquid feed condition.

The operation indicator 92 is projected in the shape of a small-height truncated cone as a whole, and has four LEDs in 90° divisions in plan view. Each of the LEDs is of two-color type which can be turned on in red and green. The operation indicator 92 is so designed that the four LEDs are repeatedly turned on in green sequentially in clockwise order, in an image of a rotary member being rotated, according to the liquid feed condition. The operation indicator 92 blinks in red when any of various unusual conditions is detected, for example, when it is decided that occlusion of the tube 22 is generated. The operation indicator 92, being projected in shape, is excellent in visibility not only from above but also from lateral sides.

The occlusion pressure monitor 94 has a configuration in which a closed state of the tube 22 decided from the pressure generated inside the syringe outer cylinder 18 is indicated by four symbols 94a, 94b, 94c and 94d which can be turned on. The symbols 94a to 94d are arranged sequentially from the left side toward the right side. When the pressure is sufficiently low, all the symbols 94a-94d are turned off. As the pressure increases, the symbols 94a, 94b and 94c are sequentially turned on. When a prescribed pressure is reached starting from the condition where the symbols 94a to 94c are turned on, it is decided that occlusion of the tube 22 is generated and the symbol 94d is made to blink, with the symbols 94a to 94c kept turned on.

The residual amount detection LED 96 is made to blink as an alarm when it is decided that the residual amount of liquid in the syringe 12 is reduced. The residual amount of liquid in the syringe may be determined from the position of the slider 60.

The operating part 32 has the liquid crystal monitor 102, function switches 104a, 104b and 104c, and an integration-clear switch 106 as information input/output means 100.

The liquid crystal monitor 102 is located at a position promising good visibility, and has an appropriately large area suited to visual check. The liquid crystal monitor 102 is of a dot matrix type, which can arbitrarily display various symbols and character strings notwithstanding its simplicity, inexpensiveness and low power consumption. Naturally, the liquid crystal display 102 can make a display in any of different languages according to the destination of the system. The liquid crystal display 102 has a horizontally (in plan view) elongated shape according to the operating part 32, is composed, for example, of 240 × 64 dots, and is measuring 99.5 mm by 28.0 mm. The liquid crystal display 102 has a backlight function which can be turned on and off in red and white.

The integration-clear switch 106 is a switch for clearing the value of integrated liquid feed amount being currently displayed on the liquid crystal monitor 102. In order to clearly show the relationship between the integration-clear switch 106 and the liquid crystal display 102, a line interconnecting both of them is printed.

The function switches 104a to 104c are arranged at regular intervals on the lower side (in plan view) of the liquid crystal monitor 102, and correspond respectively to three choices displayed in a lower area (in plan view) of the liquid crystal monitor 102.

As shown in FIG. 5, the syringe pump 10 has a computer (control unit) 110 for controlling the whole part thereof. The computer 110 is, for example, a one-chip microcomputer, having a ROM 112, a RAM 114, the nonvolatile memory (flash memory or the like) 116, and a clock 118. The computer 110 has an interface (not shown), and executes input/output processing for each of the above-mentioned sensors, switches and lamps. The clock 118 is so designed that the current time can be corrected by a predetermined operation, and it is able to acquire the current time, to measure the lapse of time during a predetermined operation, and to measure reference time for speed control.

The syringe pump 10 has the power source plug socket 39 which is supplied with external AC (or DC) power and which supplies electric power to each component through a power source part 120, the lithium ion battery (secondary battery) 122 which is charged from the power source plug socket 39 through the power source part 120 and is capable of supplying electric power to each component, and the external power source detecting part 125 for detecting the state of connection of an external power source to the power source plug socket 39. The power source part 120 produces a stable DC power source from AC, and supplies it to each component. With the lithium ion battery 122, the syringe pump 10 can be operated for a fairly long time. The power source part 120 may not necessarily be incorporated in the syringe pump 10; for example, it may be provided externally as a DC adapter.

When it is decided based on a signal obtained from the external power source detecting part 125 that no external power source is being connected, the computer 110 displays on the liquid crystal monitor 102 information for prompting the operator to connect an external power source (see FIG. 13), and turn off the external power source lamp 82.

In the syringe pump 10, the driving rod 63 for sliding the slider 60 is driven by a motor 128 provided inside the housing 11, and the drive amount is detected by an encoder 130. A driving current of the motor 128 is detected by a current sensor 132. The motor 128 is driven through a motor driver 134 under the operation of the computer 110. The computer 110 detects the pressure on the slider 60 by a strain gauge, and controls the turning-on/off of the operation indicator 92 and the occlusion pressure monitor 94.

The displacement amount of the lift rod 52 is detected by the clamp sensor 136, the operating condition of the lock lever 70 is detected by a lock sensor 138, and the respective signals are supplied to the computer 110, where the clamping of the syringe outer cylinder 18 with the clamp 50 and the fixation of the slider 60 are decided. The computer 110 turns off the clamp check LED 58 when the detection switches 40a and 40b are ON and it is decided that the syringe outer cylinder 18 is clamped with the clamp 50. The computer 110 turns off the slider check LED 72 when the plunger detection switch 64 is ON and it is decided that the slider 60 is fixed.

The computer 110 controls the display on the liquid crystal monitor (display unit) 102 through a liquid crystal driver 140, and turns on the backlight 142 with a predetermined timing. The buzzer 144, a speaker 146, a communication port 148 and a nurse call output pat 149 are connected to the computer 110, in addition to the liquid crystal monitor 102 as general-purpose output means. The communication port 148 is capable of input/output of information to and from a predetermined external computer. In the following description, it is assumed that the buzzer 144 is operated to generate a sound as a predetermined alarm or caution signal. However, a system may be adopted in which guidance is given by a sound or voice through the speaker 146.

Now, operation of the liquid crystal monitor 102 in the syringe pump 10 configured as above will be described below. In the syringe pump according to the present embodiment, information which is detailed and diverse and which can be easily understood even by persons unaccustomed to operations of the syringe pump 10 can be displayed with symbols and/or character strings on the liquid crystal monitor 102 according to the operating conditions. For instance, (1) display at the time of an operation of setting the syringe 12 to the syringe pump 10, (2) display related to the power source condition, (3) display at the time of generation of an alarm, and (4) display at the time of carrying out a prescribed inspection operation, can be performed on the liquid crystal monitor 102. First, the display at the time of an operation of setting the syringe 12 to the syringe pump 10 in item (1) above will be described.

When the power source switch 80 is depressed by an operator to start the syringe pump 10, the computer 110 executes a predetermined initialization process, thereafter prompts three operations for setting the syringe 12, checks each of the operations, and displays the check results. The three operations for setting the syringe 12 are the following a-operation, b-operation and c-operation (the a-operation, b-operation and c-operation are operation names for convenience) in a general and preferable order of operations. As for the order of the three operations for setting the syringe 12, for example, b-operation and c-operation may be carried out in the reverse order.

As shown in FIG. 6, the a-operation is an operation of mounting the syringe outer cylinder 18 of the syringe 12 onto the syringe mounting part 34, with the outer cylinder flange 20 set in the setting groove 42.

As shown in FIG. 7, the b-operation is an operation of sliding the slider 60 to an appropriate position, setting the plunger flange 28 onto the slider 60 by the levers 66, and fixing the slider 60. Strictly speaking, the b-operation is composed of two operations, namely, an operation of holding the plunger flange 28 onto the slider 60 and an operation of fixing the slider 60. On the basis of the operator's operation, however, the b-operation is composed only of an operation of the lock lever 70, and, therefore, it is appropriate to classify the b-operation as a single operation.

As shown in FIG. 8, the c-operation is an operation of appropriately drawing up the clamp 50, turning the rotating lever 54 by 90° clockwise and then lowering it, and fixing the upper surface of the syringe outer cylinder 18 by holding down it with the rotating lever 54.

Until the time when the syringe normal mount state is attained, the computer 110 performs a display (described later) on the liquid crystal monitor 102, causes the backlight 142 of the liquid crystal monitor 102 to blink alternately in red and in white, causes the corresponding clamp check LED 58 and slider check LED 72 to blink, and operates the buzzer 144 to intermittently generate a sound, thereby promoting the operator to set the syringe 12. When the backlight 142 is turned on or made to blink in red, there is obtained a mental attention exciting effect on the operator. Where the backlight 142 is of a red type, simple blinking of it suffices.

In relation to the setting of the syringe 12, further, the computer 110 monitors the detection switches 40a and 40b, the plunger detection switch 64, the clamp sensor 136 and the lock sensor 138, and displays on the liquid crystal monitor 102 guidance related to operations according to the situation. Until the syringe normal mount state is reached, character information is displayed on a character information display section 102a constituting a roughly right half (in plan view) of the liquid crystal monitor 102, and schematic graphics are displayed on a graphic display section 102b constituting the remaining roughly left half of the liquid crystal monitor 102. In the character information display section 102a, black characters are displayed against a white background, whereas in the graphic display section 102b, white graphics are displayed against a black background, whereby division between the information at the left and the information at the right is make clear. In the following drawings, the black background areas are hatched. The division between the character information display section 102a and the graphic display section 102b is a division for convenience, and the sizes and display forms in these sections may be changed according to the conditions.

First, in an initial condition where none of the a-operation, b-operation and c-operation is carried out based on sensor information (a condition where detection information from sensors is not inputted to the computer 110), the computer 110 displays on the liquid crystal monitor 102 an initial state screen 200a shown in FIG. 9A and the first syringe normal mount state screen 200b shown in FIG. 9B, repeatedly at an appropriate interval (for example, at a cycle time of 2 seconds).

The initial state screen 200a shown in FIG. 9A has three rows in the character information display section 102a. A character string "Set the flange" corresponding to the a-operation is displayed in the top row, a character string "Set the plunger" corresponding to the b-operation is displayed in the middle row, and a character string "Lower the clamp" corresponding to the c-operation is displayed in the bottom row, thereby indicating that each of these operations has not yet been carried out.

In the initial state screen 200a, schematic graphics of the plunger 16, the syringe outer cylinder 18, the outer cylinder flange 20, the tube 22, the gasket 26, the plunger flange 28, the setting groove 42, the clamp 50, the lift rod 52, the rotating lever 54, the slider 60, the levers 66 and the lock lever 70 are displayed in the graphic display section 102b. In the graphic display section 102b of the initial state screen 200a, the outer cylinder flange 20 is displayed in a solid-black pattern, thereby showing that the a-operation has not yet been carried out. In addition, the plunge flange 28 is displayed in a solid-black pattern and the slider 60 is displayed at the right end, thereby to show that the b-operations has not yet been conducted. Further, the rotating lever 54 is displayed as oriented horizontally (in plan view), thereby showing that the c-operation has not yet been performed. These portions are connected by arrowed lines respectively to the corresponding character strings in the three-row display area in the character information display section 102a, whereby the corresponding relations can be clearly understood.

The first syringe normal mount state screen 200b is the same as the initial state screen 200a in the display in the character information display section 102a. The graphic display section 102b in the first syringe normal mount state screen 200b displays the schematic graphics of the same elements as those in the initial state screen 200a, but it shows the final syringe normal mount state, and it differs from that in the initial state screen 200a in the following points. Firstly, the outer cylinder flange 20 inside the setting groove 42 is displayed in white, thereby prompting the operator to mount the outer cylinder flange 20 into the setting groove 42 as the a-operation. Secondly, the plunger flange 28 is displayed in white, the slider 60 is displaced slightly to the left and the plunger flange 28 is roughly surrounded by the levers 66, thereby prompting for the b-operation. Thirdly, the rotating lever 54 is displayed as oriented vertically (in plan view), thereby prompting for the c-operation.

Thus, in the liquid crystal monitor 102, simultaneously with the display of the character information, the object to be operated is displayed in a graphic which is changed so as to indicate the contents of the operation. Therefore, the operation of setting the syringe 12 can be carried out, without referring to a manual, even by a person who does not understand the method of mounting the syringe 12 or operations of the clamp 50 and the slider 60 and the like or who is not frequently engaged in the operation.

Incidentally, referring to FIG. 9A alone, it may seem difficult to understand which of the components in the graphic display section 102b is yet to be operated. In practice, however, the initial state screen of FIG. 9A and the first syringe normal mount state screen of FIG. 9B are displayed alternately and repeatedly at an appropriate interval, and, therefore, the different points between the two states can be grasped clearly and easily.

Next, the operator carries out the a-operation, based on the initial state screen 200a and the first syringe normal mount state screen 200b and based on "Set the flange" displayed in the top row (in plan view) in the character information display section 102a. Since this character information is displayed in the top row, it is easily understood that it is preferable to carry out this operation first.

With the a-operation carried out properly, the detection switches 40a and 40b are turned ON, and detection information is inputted to the computer 110. As a result, the computer 110 decides that the a-operation has been conducted properly. As a subsequent step, the computer 110 displays a flange set state screen 202a shown in FIG. 10A and a second syringe normal mount state screen 202b shown in FIG. 10B, alternately and repeatedly at an appropriate interval. When at least one of the detection switches 40a and 40b is kept in an OFF state, detection information is not inputted therefrom to the computer 110. In this case, the computer 110 decides that the a-operation has not yet been completed or has been carried out improperly, and the control does not proceed to the display of the flange set state screen 202a and the second syringe normal mount state screen 202b.

The flange set state screen 202a shown in FIG. 10A differs from the initial state screen 200a (see FIG. 9A) in that the fact that the a-operation has been finished is displayed in characters and in graphic. Specifically, in the character information display section 102a of the flange set state screen 202a, the character string "Set the flange" in the top row has been removed from display. In the graphic display section 102b of the flange set state screen 202a, the outer cylinder flange 20 which has been displayed in the solid-black pattern in the initial state screen 200a is now displayed in white. These display conditions show that the a-operation has been finished. On the other hand, the b-operation and the c-operation are yet to be carried out. Therefore, in the character information display section 102a, the character string "Set the plunger" in the middle row and the character string "Lower the clamp" in the bottom row are left in display. Besides, in the graphic display section 102b, the rotating lever 54 is displayed as oriented horizontally (in plan view), the plunger flange 28 is displayed in a solid-black pattern, and the slider 60 is displayed at the right end.

In the second syringe normal mount state screen 202b shown in FIG. 10B, the character information display section 102a is the same as that in the flange set state screen 202a. In the second syringe normal mount state screen 202b, the graphic display section 102b is the same as that in the first syringe normal mount state screen 200b, showing the final syringe normal mount state.

Thus, the flange set state screen 202a and the second syringe normal mount state screen 202b are displayed alternately, whereby the operator is permitted to easily grasp that the a-operation has been completed properly and that the b-operation and the c-operation are yet to be finished.

Next, the operator performs the b-operation, based on the flange set state screen 202a and the second syringe normal mount state screen 202b and based on "Set the plunger" displayed in the middle row in the character information display section 102a.

With the b-operation carried out properly, the plunger detection switch 64 is turned ON, the detection information is inputted therefrom, and a signal from the lock sensor 138 indicates a fixed state of the slider 60. As a result, the computer 110 decides that the b-operation has been conducted properly. As a subsequent step, the computer 110 displays a slider set state screen 204a shown in FIG. 11A and a third syringe normal mount state screen 204b shown in FIG. 11B, alternately and repeatedly at an appropriate interval. In addition, when it is decided that the b-operation has been performed properly, the slider check LED 72 is transferred from a blinking state to an off state.

When the plunger detection switch 64 is OFF or the signal from the lock sensor 138 is indicating a released state of the slider 60, the detection information is not inputted therefrom to the computer 110. Therefore, the computer 110 decides that the b-operation has not yet been finished or has been carried out improperly. In this case, the control does not proceeds to the display of the slider set state screen 204a and the third syringe normal mount state screen 204b.

A slider set state screen 204a shown in FIG. 11A differs from the initial state screen 200a (see FIG. 9A) in that the fact that the a-operation has been finished is displayed in characters and in graphic. Specifically, in the character information display section 102a of the slider set state screen 204a, the character string "Set the plunger" in the top row has been removed from display. Besides, in the graphic display section 102b of the slider set state screen 204a, the plunger flange 28 which has been displayed in the solid-black pattern in the initial state screen 200a is now represented in white, the slider 60 which has been displayed at the right end before is now displaced slightly to the left, and the plunger flange 28 is roughly surrounded by the levers 66. These displays show that the b-operation has been finished. Since the c-operation is yet to be performed, the character string "Lower the clamp" in the bottom row in the character information display section 102a is left in display, and the rotating lever 54 in the graphic display section 102b is displayed as oriented horizontally (in plan view).

In the third syringe normal mount state screen 204b shown in FIG. 11B, the character information display section 102a is the same as that in the slider set state screen 204a. The graphic display section 102b in the third syringe normal mount state screen 204b is the same as that in the first syringe normal mount state screen 200b, showing the final syringe normal mount state.

Thus, the slider set state screen 204a and the third syringe normal mount state screen 204b are displayed alternately, whereby the operator is permitted to easily grasp that the a-operation and the b-operation have been completed properly and that the c-operation is yet to be finished.

Next, the operator carries out the c-operation, based on the slider set state screen 204a and the third syringe normal mount state screen 204b and based on "Lower the clamp" displayed in the bottom row in the character information display section 102a.

With the c-operation conducted properly, detection information from the clamp sensor 136 is inputted to the computer 110, so that it is decided that the syringe outer cylinder 18 has been fixed by the clamp 50. In this case, whether the c-operation has been carried out properly or not is decided by the computer 110, based on that the rotating lever 54 of the clamp 50 is oriented vertically (in plan view) and on that the advance/retraction amount of the clamp 50 is roughly coincident with one of a plurality of prescribed values. The prescribed values are set, based on the outside diameters of a plurality of syringes 12 which are applicable here.

When it is decided that the c-operation has been performed properly, display of a setting-completed screen 206 shown in FIG. 12 is carried out as a subsequent step. Besides, when it is decided that the a-operation and the b-operation have been carried out properly, the clamp check LED 58 is transferred from a blinking state to an off state.

When the rotating lever 54 is not oriented vertically (in plan view) or the advance/retraction amount of the clamp 50 is deviated from the plurality of prescribed values, it shows that the c-operation has not yet been finished or has been conducted improperly, so that the control does not proceed to the display of the setting-completed screen 206. In addition, in the case where the condition in which the advance/retraction amount of the clamp 50 is deviated from the plurality of prescribed values although the rotating lever 54 is oriented vertically (in plan view) has continued for a predetermined time, it is decided that a syringe not conforming to the prescribed specifications has been mounted, and the decision may be displayed on the liquid crystal monitor 102 to excite the operator's attention.

As shown in FIG. 12, in the setting-completed screen 206, a character string "Setting completed" is displayed in large size in the character information display section 102a, showing that the syringe normal mount state is attained. The graphic display section 102b in the setting-completed screen 206 is the same as that in the first syringe normal mount state screen 200b. However, this display of graphics is not alternate with display of other graphics, and, accordingly, the attainment of the syringe normal mount state can be recognized on a graphical basis, as well.

Thus, when the operation of mounting the syringe 12 is finished normally and the syringe normal mount state is obtained, the normal completion is clearly displayed in graphics and (or) in character information. Consequently, checking of whether the operation has been conducted properly or not is more facilitated, and the operator can understand that there is no need for other operations. This enhances the operator's sense of security.

When the syringe normal mount state is attained, the backlight 142 of the liquid crystal monitor 102 is turned on in white, whereas the corresponding clamp check LED 58 and slider check LED 72 are turned off, and the buzzer 144 is stopped operating.

Thereafter, the liquid feed rate can be set by operating the setting dial 86, and liquid feed can be started by depressing the start switch 90a.

During the liquid feeding operation, an operation screen 210 shown in FIG. 13 is displayed on the liquid crystal monitor 102. A power source symbol 212 at the upper left end of the operation screen 210 indicates the charged state of the lithium ion battery 122, a syringe column 214 at the right thereof indicates the kind of the syringe 12 distinguished by the signal from the clamp sensor 136 at the clamp 50, and a occlusion column 216 at the upper middle indicates the level of occlusion of the tube 22. In the syringe column 214, the manufacturer and the size of the syringe 12 are displayed.

On the operation screen 210, an integrated amount display section 218 is provided in a right side area, and an information display section 230 is provided in a lower area. In the information display section 230, various kinds of information are displayed in character strings according to the conditions at a given time, and key display parts 232a, 232b, 232c indicative of the functions of the function switches 104a to 104c at the given time are displayed. In the example shown in FIG. 13, no function is allocated to the function switch 104c at the given time, so that a key display part 232c is not displayed but is implied by imaginary lines.

As above-described, in the syringe pump 10 according to the present embodiment, the liquid crystal monitor 102 under the operation of the computer 110 displays information about the a-operation, b-operation and c-operation related to the setting of the syringe 12; specifically, the liquid crystal monitor 102 displays the graphic of the syringe 12, the graphic of the slider 60 and the graphic of the clamp 50, with changes (change between white display and solid-black display and/or change of displaying positions) of those portions of the graphics which correspond to the operations. Thus, the operations are displayed by use of graphics the state of which is changed before and after the relevant operation, whereby it is made easier to grasp what operation should be made in setting the syringe 12. In other words, the syringe 12 can be set, without referring to a manual, even by a person who does not understand the method of mounting the syringe 12 or operations of the clamp 50 and the slide 60 and the like or who is not frequently engaged in the operations.

The changing graphics display the states before and after each of the a-operation, b-operation and c-operation, alternately and repeatedly at a predetermined interval. This ensures that the differences between the state before a given operation and the state after the operation can be grasped clearly and easily, so that the operator can more easily understand what operation should be done.

Besides, in the syringe pump 10, when each of the a-operation, b-operation and c-operation has been carried out properly, the corresponding one of the graphic of the syringe outer cylinder 18 in the syringe 12 and the graphic of the slider 60 and the graphic of the clamp 50 is displayed as a graphic indicative of completion of the operation, whereby proper completion of each operation can be confirmed on a procedure basis. This permits not only a person unaccustomed to the operations of the syringe pump 10 but also a person accustomed to the operations to more easily check whether each operation has been properly conducted or not.

In addition, it is possible to grasp which one or ones of the a-operation, b-operation and c-operation have been finished and which one of ones of these operations have not yet been finished or have been made improperly (operation mistake).

Further, the liquid crystal monitor 102 has the character information display section 102a, wherein the pieces of character information corresponding respectively to the a-operation, b-operation and c-operation are displayed, and, when a given operation has been conducted, the corresponding piece of character information is removed from display. With the character information thus displayed in addition to the graphics, the procedure of operations and whether each operation has been carried out properly or not can be checked more easily.

Besides, when all of the a-operation, b-operation and c-operation have been completed, the fact that all the operations have been finished correctly is clearly displayed with the setting-completed screen 206, whereby the checking of whether the operations have been carried out properly or not is more facilitated, which enhances the operator's feeling of security.

Now, the display related to the power source condition in the above-mentioned item (2) will be described below.

The syringe pump 10 is so designed that the operation thereof can be continued by use of the lithium ion battery 122 incorporated therein, for a certain period of time, in order to ensures that even upon stop of power supply from an external power source, a predetermined coping with the situation can be made. Basically, however, it is desirable to securely supply the syringe pump 10 with electric power from an external power source.

In view of this, the computer 110 functions as follows. When it is decided, based on a signal obtained from the external power source detecting part 125, that electric power is not being supplied from an external power source, the computer 110 displays information for promoting for connection of an external power source, specifically, displays a character string "Please connect the AC cable" in the information display section 230 on the liquid crystal monitor 102. In addition, the computer 110 causes the external power source lamp 82 and the operation indicator 92 to blink. As a result, a situation in which the syringe pump 10 is left in the state of being supplied with electric power from the lithium ion battery 122 (namely, in the discharging state of the lithium ion battery 122) is obviated more securely, and the syringe pump 10 is supplied with power from an external power source assuredly.

In addition, in the case where it is decided that the AC cable 39a is pulled off from the power source plug socket 39 (inclusive of the case where the AC cable 39a has been pulled off at the time of starting the syringe pump 10), the buzzer 144 is operated for a short time, and the backlight 142 is turned on in red for a predetermined time. This permits the operator to more securely grasp that the AC cable 39a has been pulled off. Particularly, the situation of disengagement of the AC cable 39a can be confirmed by way of the character information in the information display section 230 after the excitation of attention by the buzzer 144 and the backlight 142. This naturally is preferable.

Further, by changing the form of the power source symbol 212, the residual charge amount of the lithium ion battery 122 at a given time and the state of charging are displayed. Specifically, the basic shape of the power source symbol 212 is a dry-cell shape elongated horizontally (in plan view), and a maximum of five bars extending vertically (in plan view) are displayed according to the residual charge amount.

In the case where there is no vertical bar in the power source symbol 212 as shown in FIG. 14A, the symbol represents that the lithium ion battery 122 has been fully discharged and has no residual charge therein. In the case where three vertical bars are present in the power source symbol 212 as shown in FIG. 14B, the symbol represents that the residual charge amount of the lithium ion battery 122 is about one half the full charge. In the case where five vertical bars are present in the power source symbol 212 as shown in FIG. 14C, the symbol represents that the lithium ion battery 122 is in a fully charged state. Consequently, the operator can easily check the residual charge amount of the lithium ion battery.

The power source symbol 212 is displayed continuously in the case where power supply from an external power source is absent. In this case, the extent of lowering in the residual charge amount can be easily checked, based on a gradual decrease in the number of the vertical bars.

In the case where power supply from an external power source is present and where the lithium ion battery 122 is not in a fully charged state, the lithium ion battery 122 is charged by the electric power from the external power source. In this case, for clearly indicating that charging is under way, the power source symbol 212 indicative of the charged state at a given time (namely, one of the symbols shown in FIGS. 14A to 14C) and a charging-now display symbol 212a shown in FIG. 15 are displayed alternately and repeatedly at an appropriate interval. Consequently, the operator can easily confirm the fact that normal charging of the lithium ion battery 122 is being performed, and the residual charge amount at the given time.

Now, the display at the time of generation of an alarm in the above-mentioned item (3) will be described below.

During liquid feeding by use of the syringe pump 10, detection of various unusual conditions is performed. In this connection, it is desirable that, upon detection of an unusual condition, the kind of the unusual condition can be easily grasped.

In view of this, the computer 110 monitors signals from various sensors during liquid feed or during predetermined standby time, decides the presence/absence of an unusual condition(s), and displays the kind(s) of the unusual condition(s) generated on the liquid crystal monitor 102.

For example, in the case where the detection switch 40a or 40b is turned OFF or the signal from the clamp sensor 136 is changed, during liquid feed, it can be decided that an unusual condition of disengagement of the syringe outer cylinder 18 from the syringe mounting part 34 has occurred. Therefore, as shown in FIG. 16, a large-sized character string "Syringe disengaged" is displayed in void black characters in a roughly upper half area; in addition, an exclamation mark "!" is displayed in a triangle. In this manner, the generation of an unusual condition and the kind of the unusual condition are displayed, and the operator's attention is excited.

In the information display section 230 on the liquid crystal monitor 102, the item to be conducted by the operator is displayed; for example, a character string "Please check the set state of the syringe" is displayed. Naturally, a more specific item(s) corresponding to the situation may be displayed, depending on the kind of the unusual condition. As to the kind of the unusual condition, for example, a display may be made according to the graphics shown in FIGS. 9A to 11B.

In the key display part 232c, a character string "OK" is displayed as a function of the function switch 104c. Thus, a function for permitting the operator to recognize the item shown in the information display section 230 and for informing the computer 110 that a predetermined process corresponding to the unusual condition has been completed is allocated to the function switch 104c.

In addition, simultaneously, the backlight 142 is turned on continuously in red, the corresponding clamp check LED 58 and operation indicator 92 are made to blink, and further the buzzer 144 is operated continuously, whereby the operator can be securely informed of generation of an unusual condition. In this case, the sounding of the buzzer 144 can be stopped by depressing a predetermined switch. This ensures that the operator can cope with the situation composedly in a calm atmosphere, and that the patient is prevented from needlessly becoming anxious.

After the buzzer 144 is stopped by depressing the predetermined switch and a predetermined process of coping with the unusual condition is conducted and the function switch 104c is depressed, a first post-unusual-disposal screen 234a shown in FIG. 17A and a second post-unusual-disposal screen 234a shown in FIG. 17B are displayed alternately and repeatedly at an appropriate interval. In the information display section 230 on the first post-unusual-disposal screen 234a, a character string "Stopped because of disengagement of syringe" is displayed to indicate the cause of the unusual condition. In the information display section 230 on the second post-unusual-disposal screen 234b, a character string "After confirmation, please depress START" is displayed as a prompt for resumption.

During when the first post-unusual-disposal screen 234a and the second post-unusual-disposal screen 234b are displayed, the backlight 142 is made to blink in red, the clamp check LED 58 is make to blink (when the unusual condition continues) or is turned off (when the unusual condition is cleared) according to the situation, and the buzzer 144 is kept stopped. It is to be noted, however, that in order to prevent the syringe pump 10 from being left in the post-unusual-disposal condition for a long time, the buzzer 144 is again operated after a predetermined lapse of time (for example, after two minutes), thereby prompting for resuming of liquid feed, or it is displayed that the syringe pump 10 is being left in the post-unusual-disposal condition.

The liquid feed can be resumed by depressing the start switch 90a according to the display on the second post-unusual-disposal screen 234b.

Thus, in the syringe pump 10, upon detection of an unusual condition, the kind of the unusual condition is displayed with graphics and (or) character information, whereby the operator is permitted to easily grasp the kind.

Now, the display at the time of carrying out a prescribed inspection operation, as specified in the above-mentioned item (4), will be described below.

In application of the syringe pump 10, generally, routine inspections according to predetermined standards are made compulsory. Examples of the compulsory inspection include inspection of the accuracy of flow rate during liquid feed, inspection of an occlusion-detecting function, and inspection of the capacity of the lithium ion battery 122. These routine inspections are carried out in a plurality of procedures for a plurality of items. If such a plurality of procedures can be carried out without a manual, the burden on the operator is alleviated, which is desirable.

In view of this, for example, in the case of performing the inspection of the accuracy of flow rate during liquid feed, the computer 110 displays on the liquid crystal monitor 102 a flow rate accuracy inspection display 240 shown in FIG. 18.

In inspecting the accuracy of flow rate, a syringe 12 filled with a medicinal liquid is set, setting of a liquid feed rate is conducted, actual liquid feed is conducted, and whether the liquid feed is proper or not is decided based on the liquid feed rate over a predetermined inspection time.

In the flow rate accuracy inspection display 240, a procedure to be carried out next during inspection is displayed in the information display section 230; for example, a character string "Please set the flow rate and time, and start the liquid feed" is displayed. Characters "back" and a return mark are displayed in the key display part 232a, which shows that a function of returning the inspection procedure by one step is allocated to the corresponding function switch 104a. A downwardly (in plan view) pointed triangle mark is displayed in the key display part 232c, showing that a function of advancing the inspection procedure by one step is allocated to the corresponding function switch 104c.

On the upper side (in plan view) of these display parts, an elapsed-time display part 242 is provided, and the lapse of inspection time is displayed under the operation of the clock 118. When a predetermined inspection time ends and the display in the elapsed-time display part 242 becomes "0," the liquid feed is automatically stopped. Thus, the inspection is simple.

As above-mentioned, in the syringe pump 10, at the time of performing a prescribed inspection operation, specific operations to be carried out according to a procedure for the inspection operation are displayed on the liquid crystal monitor 102. Therefore, the inspection operation can be carried out without referring to a manual, whereby the burden on the operator is reduced. As for the procedure with the lapse of time, the elapsed time is displayed in the elapsed-time display part 242; therefore, it is unnecessary to separately prepare a stopwatch or to manually perform a liquid feed stopping operation while checking a stopwatch.

In using the syringe pump 10, naturally, the inspections may be carried out while referring to a manual.

The syringe pump according to the present invention is not limited to the above-described embodiment, and, naturally, various configurations can be adopted without departure from the gist of the invention.

## Claims

1. A syringe pump (10), comprising:
a syringe mounting part (34) on which to mount an outer cylinder (18) of a syringe (12a);
a slider (60) for holding a plunger (16) of the syringe (12a) mounted on the syringe mounting part (34);
a clamp (50) which contacts an upper surface of the outer cylinder (18) mounted on the syringe mounting part (34) and which thereby fixes the outer cylinder (18);
an outer cylinder detecting part (40a, 40b) for detecting that the outer cylinder (18) is mounted on the syringe mounting part (34);
a plunger detecting part (64) for detecting that the plunger (16) is held by the slider (60);
a clamp detecting part (136) for detecting that the outer cylinder (18) is fixed by the clamp (50);
a display unit (102) for displaying predetermined information; and
a control unit (110) for controlling the display of the display unit (102); whereby
the control unit (110) is connected to the outer cylinder detecting part (40a, 40b), the plunger detecting part (64) and the clamp detecting part (136), to produce detection information therefrom, and is inputted with the detection information; wherein
the control unit (110) is configured to perform a control, based on the detection information, for displaying guidance in a character information display section (102a) and/or graphic display section (102b) showing respective changes between the states of the syringe pump before and after the operation of mounting the outer cylinder (18) onto the syringe mounting part (34), the operation of holding the plunger (16) by the slider (60) and the operation of fixing the outer cylinder (18) by the clamp (50).

2. The syringe pump (10) according to claim 1, wherein the display unit (102) is configured to display graphics showing respective states before and after the operations, alternately and repeatedly at a predetermined interval.

3. The syringe pump (10) according to claim 1 or 2, wherein the display unit (102) is configured to display a graphic showing the state after completion of the corresponding operation, when the detection information from the outer cylinder detecting part (40a, 40b), the plunger detecting part (64), and the clamp detecting part (136) is inputted to the control unit (110).

4. The syringe pump (10) according to any one of claims 1 to 3, wherein the control unit (110), upon deciding that an unusual condition is generated, displays information indicative of the kind of the unusual condition on the display unit (102).

5. The syringe pump (10) according to claim 4, wherein after all of pieces of detection information from the outer cylinder detecting part (40a, 40b), the plunger detecting part (64), and the clamp detecting part (136) are inputted to the control unit (110) and when at least one of the pieces of detection information is changed to an non-detection information, the display unit (102) displays information indicative of the kind of an unusual condition corresponding to the change.

6. The syringe pump (10) according to claim 4 or 5, wherein the display unit (102) has a backlight (142) capable of being turned on and off, and causes the backlight (142) to blink when it is decided by the control unit (110) that an unusual condition is generated.

7. The syringe pump (10) according to any one of claims 1 to 6, further comprising:
a power source terminal (39) through which external electric power is supplied and fed to each component;
a secondary battery (122) which is charged through the power source terminal (39) and is capable of feeding electric power to each component; and
an external power source detecting part (125) for detecting the state of connection of external electric power to the power source terminal (39);
wherein the control unit (110) displays on the display unit (102) information for prompting for connection of the external power source when it is decided, based on a signal from the external power source detecting part (125), that the external power source is not being connected.

8. The syringe pump (10) according to any one of claims 1 to 7, wherein at the time of performing a prescribed inspection operation, the control unit (110) displays on the display unit (102) specific operations according to a procedure for the inspection operation.

## Patentansprüche

1. Injektionsspritzenpumpe (10), die Folgendes enthält:
einen Injektionsspritzenbefestigungsteil (34), an dem ein Außenzylinder (18) einer Injektionsspritze (12a) angebracht werden soll;
einen Schieberegler (60), um einen Kolben (16) der Injektionsspritze (12a), die am Injektionsspritzenbefestigungsteil (34) angebracht ist, zu halten;
eine Klemme (50), die eine Oberseite des Außenzylinders (18), der am Injektionsspritzenbefestigungsteil (34) angebracht ist, kontaktiert und dadurch den Außenzylinder (18) befestigt;
einen Außenzylinderdetektionsteil (40a, 40b), um zu detektieren, dass der Außenzylinder (18) am Injektionsspritzenbefestigungsteil (34) angebracht ist;
einen Kolbendetektionsteil (64), um zu detektieren, dass der Kolben (16) durch den Schieberegler (60) gehalten wird;
einen Klemmendetektionsteil (136), um zu detektieren, dass der Außenzylinder (18) durch die Klemme (50) befestigt ist;
eine Anzeigeeinheit (102), um vorgegebene Informationen anzuzeigen; und
eine Steuereinheit (110), um die Anzeige der Anzeigeeinheit (102) zu steuern; wobei
die Steuereinheit (110) mit dem Außenzylinderdetektionsteil (40a, 40b), dem Kolbendetektionsteil (64) und dem Klemmendetektionsteil (136) verbunden ist, um hiervon Detektionsinformationen zu erzeugen, und die Detektionsinformationen einliest; wobei
die Steuereinheit (110) konfiguriert ist, eine Steuerung auf der Grundlage der Detektionsinformationen durchzuführen, um eine Führung in einem Zeicheninformationsanzeigeabschnitt (102a) und/oder einem graphischen Anzeigeabschnitt (102b) anzuzeigen, die entsprechenden Änderungen zwischen den Zuständen der Injektionsspritzenpumpe vor und nach dem Vorgang des Anbringens des Außenzylinders (18) am Injektionsspritzenbefestigungsteil (34), dem Vorgang des Haltens des Kolbens (16) durch den Schieberegler (60) und dem Vorgang des Befestigens des Außenzylinders (18) durch die Klemme (50) zeigt.

2. Injektionsspritzenpumpe (10) nach Anspruch 1, wobei die Anzeigeeinheit (102) konfiguriert ist, Graphiken, die entsprechende Zustände vor und nach den Vorgängen zeigen, abwechselnd und in einem vorgegebenen Intervall wiederholt anzuzeigen.

3. Injektionsspritzenpumpe (10) nach Anspruch 1 oder 2, wobei die Anzeigeeinheit (102) konfiguriert ist, eine Graphik anzuzeigen, die den Zustand nach dem Abschließen des entsprechenden Vorgangs zeigt, wenn die Detektionsinformationen vom Außenzylinderdetektionsteil (40a, 40b), vom Kolbendetektionsteil (64) und vom Klemmendetektionsteil (136) in die Steuereinheit (110) eingegeben werden.

4. Injektionsspritzenpumpe (10) nach einem der Ansprüche 1 bis 3, wobei die Steuereinheit (110) aufgrund des Entscheidens, dass ein außergewöhnlicher Zustand entsteht, Informationen, die die Art des außergewöhnlichen Zustands angeben, auf der Anzeigeeinheit (102) anzeigt.

5. Injektionsspritzenpumpe (10) nach Anspruch 4, wobei dann, wenn alle Detektionsinformationselemente vom Außenzylinderdetektionsteil (40a, 40b), vom Kolbendetektionsteil (64) und vom Klemmendetektionsteil (136) in die Steuereinheit (110) eingegeben worden sind und wenn mindestens eines der Detektionsinformationselemente zu einem Nicht-Detektionsinformationselement geändert wird, die Anzeigeeinheit (102) Informationen, die die Art eines der Änderung entsprechenden außergewöhnlichen Zustands angeben, anzeigt.

6. Injektionsspritzenpumpe (10) nach Anspruch 4 oder 5, wobei die Anzeigeeinheit (102) eine Hintergrundbeleuchtung (142), die ein- und ausgeschaltet werden kann, besitzt und bewirkt, dass die Hintergrundbeleuchtung (142) blinkt, wenn durch die Steuereinheit (110) entschieden wird, dass ein außergewöhnlicher Zustand entsteht.

7. Injektionsspritzenpumpe (10) nach einem der Ansprüche 1 bis 6, die ferner Folgendes enthält:
einen Leistungsquellenanschluss (39), über den externe elektrische Leistung zugeführt und in jede Komponente eingespeist wird;
eine Sekundärbatterie (122), die über den Leistungsquellenanschluss (39) geladen wird und Leistung in jede Komponente einspeisen kann; und
einen Detektionsteil (125) für eine externe Leistungsquelle, um den Zustand einer Verbindung einer externen elektrischen Leistung zum Leistungsquellenanschluss (39) zu detektieren; wobei
die Steuereinheit (110) an der Anzeigeeinheit (102) Informationen anzeigt, um zur Verbindung der externen Leistungsquelle aufzufordern, wenn auf der Grundlage eines Signals vom Detektionsteil (125) für eine externe Leistungsquelle entschieden wird, dass die externe Leistungsquelle nicht verbunden ist.

8. Injektionsspritzenpumpe (10) nach einem der Ansprüche 1 bis 7, wobei die Steuereinheit (110) zum Zeitpunkt des Durchführens eines vorgegebenen Prüfvorgangs bestimmte Vorgänge gemäß einer Prozedur für den Prüfvorgang auf der Anzeigeeinheit (102) anzeigt.

## Revendications

1. Pompe à seringue (10), comprenant :
une partie de montage de seringue (34) sur laquelle peut être monté un cylindre externe (18) d'une seringue (12a) ;
un coulisseau (60) destiné à maintenir un piston (16) de la seringue (12a) montée sur la partie de montage de seringue (34) ;
une pince (50) qui entre en contact avec une surface supérieure du cylindre externe (18) monté sur la partie de montage de seringue (34) et qui fixe ainsi le cylindre externe (18) ;
une partie de détection de cylindre externe (40a, 40b) destinée à détecter le fait que le cylindre externe (18) est monté sur la partie de montage de seringue (34) ;
une partie de détection de piston (64) destinée à détecter le fait que le piston (16) est maintenu par le coulisseau (60) ;
une partie de détection de pince (136) destinée à détecter le fait que le cylindre externe (18) est fixé par la pince (50) ;
une unité d'affichage (102) destinée à afficher des informations prédéterminées ; et
une unité de commande (110) destinée à commander l'affichage de l'unité d'affichage (102) ; de telle sorte que :
l'unité de commande (110) est raccordée à la partie de détection de cylindre externe (40a, 40b), à la partie de détection de piston (64) et à la partie de détection de pince (136), afin de produire des informations de détection de ces dernières, et reçoit les informations de détection ; dans laquelle
l'unité de commande (110) est configurée de manière à mettre en oeuvre une commande, sur la base des informations de détection, afin d'afficher des conseils sur une section d'affichage d'informations textuelles (102a) et/ou une section d'affichage graphique (102b) montrant des changements respectifs entre les états de la pompe de seringue avant et après l'opération de montage du cylindre externe (18) sur la partie de montage de seringue (34), l'opération de maintien du piston (16) par le coulisseau (60) et l'opération de fixation du cylindre externe (18) par la pince (50).

2. Pompe à seringue (10) selon la revendication 1, dans laquelle l'unité d'affichage (102) est configurée de manière à afficher des graphiques montrant des états respectifs avant et après les opérations, de manière alternée et répétée suivant un intervalle prédéterminé.

3. Pompe à seringue (10) selon la revendication 1 ou 2, dans laquelle l'unité d'affichage (102) est configurée de manière à afficher un graphique montrant l'état à l'achèvement de l'opération correspondante, lorsque les informations de détection de la partie de détection de cylindre externe (40a, 40b), la partie de détection de piston (64) et la partie de détection de pince (136) sont entrées dans l'unité de commande (110).

4. Pompe à seringue (10) selon l'une quelconque des revendications 1 à 3, dans laquelle l'unité de commande (110), lorsqu'elle détermine qu'un état inhabituel est produit, affiche des informations indicatives du type d'état inhabituel sur l'unité d'affichage (102).

5. Pompe à seringue (10) selon la revendication 4, dans laquelle, après que tous les éléments d'information de détection de la partie de détection de cylindre externe (40a, 40b), de la partie de détection de piston (64) et de la partie de détection de pince (136) ont été entrés dans l'unité de commande (110) et lorsqu'au moins l'un des éléments d'information de détection est modifié pour une information de non détection, l'unité d'affichage (102) affiche des informations indicatives du type d'état inhabituel correspondant à la modification.

6. Pompe à seringue (10) selon la revendication 4 ou 5, dans laquelle l'unité d'affichage (102) comporte un rétro-éclairage (142) pouvant être activé et désactivé, et amène le rétro-éclairage (142) à clignoter lorsqu'il est déterminé par l'unité de commande (110) qu'un état inhabituel est produit.

7. Pompe à seringue (10) selon l'une quelconque des revendications 1 à 6, comprenant, en outre :
une borne de source d'énergie (39) au moyen de laquelle de l'énergie électrique externe est fournie et délivrée à chaque composant ;
une batterie secondaire (122) qui est chargée au moyen de la borne de source d'énergie (39) et peut délivrer de l'énergie électrique à chaque composant ; et
une partie de détection de source d'énergie externe (125) destinée à détecter l'état de raccordement de l'énergie électrique externe à la borne de source d'énergie (39) ;
dans laquelle l'unité de commande (110) affiche sur l'unité d'affichage (102) des informations destinées à demander la mise en oeuvre du raccordement de la source d'énergie externe lorsqu'il est déterminé, sur la base d'un signal à partir de la partie de détection de source d'énergie externe (125), que la source d'énergie externe n'est pas raccordée.

8. Pompe à seringue (10) selon l'une quelconque des revendications 1 à 7, dans laquelle, au moment d'exécuter une opération d'inspection prédéfinie, l'unité de commande (110) affiche sur l'unité d'affichage (102) des opérations spécifiques selon une procédure de l'opération d'inspection.
